# EUROPÄISCHE PATENTANMELDUNG

(11) **EP 2 620 162 A1**
(43) Veröffentlichungstag der Anmeldung: **31.07.2013**
(21) Anmeldenummer: 12000544.2
(22) Anmeldetag: 27.01.2012
(51) Int. Cl.: A61K 47/48, A61P 1/00, A61P 31/00

(54) **Enzym-Polymer-Konjugat zur Endotoxin-Detoxifikation**

(71) Anmelder: Life Science Inkubator, 53175 Bonn (DE)
(72) Erfinder: Nowak, Götz, 99097 Erfurt (DE); Köhler, Anne, 53175 Bonn (DE); Rachow, Steffen, 53175 Bonn (DE); Stibaner,Inga, 53175 Bonn (DE); Bergner,Simone, 53119 Bonn (DE)
(74) Vertreter: von Renesse, Dorothea

(57) **Zusammenfassung**

Die Erfindung betrifft ein Enzym-Polymer-Konjugat umfassend ein Enzym und mindestens ein Polymer (Konjugatpolymer), wobei das Enzym eine a. Phosphataseaktivität oder b. Acyloxyacylhydrolase-Aktivität zeigt und in der Lage ist, Endotoxine zu entgiften, zur Verwendung in einem Verfahren zur Therapie oder Prophylaxe einer Endotoxin-vermittelten Erkrankung.

## Beschreibung

Die Erfindung betrifft ein Enzym-Polymer-Konjugat für die Endotoxin-Detoxifikation und dessen Verwendung zur Behandlung Endotoxin-vermittelter Erkrankungen.

Endotoxin-vermittelte Erkrankungen umfassen eine Vielzahl akuter oder chronischer Erkrankungen, wie beispielsweise Sepsis, Morbus Crohn, Colitis ulcerosa und verschiedene Autoimmun-Krankheiten.

Endotoxine sind Bestandteil der Zellwand gramnegativer Bakterien oder Blaualgen. Chemisch gesehen sind Endotoxine Lipopolysaccharide (LPS). Sie umfassen einen hydrophilen Polysaccharid- und einen hydrophoben Lipid-Anteil. Der auch als Lipid A bezeichnete Lipid-Anteil weist mindestens einen Phosphatrest und mindestens eine sekundär gebundene, nicht-hydroxylierte Fettsäure auf. Diese sind für die inflammatorische Wirkung des Endotoxins verantwortlich.

LPS bindet an zahlreiche Makromoleküle wie Albumin, Low density lipoprotein (LDL), High density lipoprotein (HDL) und vor allem Lipopolysaccharid-bindendes Protein (LBP/Septin). Nach primärer Bindung an LBP wird das LPS im Blut auf gelöstes CD14 übertragen, zu MD-2 transferiert und in die MD-2-"Tasche" aufgenommen. Der LPS-MD-2-Komplex bindet an den Toll-like Rezeptor 4 (TLR-4), der dabei durch Dimerisierung aktiviert wird. Die physiologische Funktion dieser TLR-Aktivierung besteht in der Induktion und Freisetzung proinflammatorischer Zytokine, wie beispielsweise dem Tumornekrosefaktor-alpha (TNFα), Interleukin (IL)-1, IL-6, IL-8 und IL-12. Weiterhin ist die Freisetzung von Stickstoffmonoxid (NO), Prostaglandinen, Leukotrienen und toxischen Sauerstoffradikalen beschrieben worden.

Zu den lebensbedrohenden Endotoxin-vermittelten Erkrankungen gehören die Sepsis und der septische Schock mit einer Inzidenz von 1-2 % in Westeuropa oder den USA. In der Todesursachenstatistik steht die Sepsis in den USA heute an dritter Stelle der infektionsbedingten Sterbefälle und an zwölfter Stelle in der Gesamtmortalität. Die Anzahl der Todesfälle nahm in den letzten zwei Jahrzehnten um 83 % zu. In Deutschland hat sich die Zahl der Todesfälle an schweren bakteriellen Infektionen seit 1980 mehr als verdreifacht. In Nordamerika sterben jährlich 150 000 Menschen an den Folgen einer Sepsis.

Die Letalität der Sepsis ist trotz Fortschritte der Medizin in den letzten Jahren mit ca. 40 % unverändert hoch. Beim septischen Schock liegt sie bei über 70 %.

Infektionen mit gram-negativen Erregern können ihren Ursprung vor allem im Darm haben. Der Darm spielt aber nicht nur als Infektionsquelle, sondern auch als Teil des Immunsystems eine große Rolle. Mehr als die Hälfte der gesamten lymphatischen Zellen des Körpers konzentrieren sich hier als darmassoziiertes lymphatisches Gewebe.

Es wird angenommen, dass den lokal in der Mikrozirkulation des Darms produzierten Entzündungsmediatoren eine entscheidende Rolle zukommt. So werden proinflammatorische Zytokine auf einen inflammatorischen Stimulus (z.B. nach Endotoxin-Durchtritt durch die "Darmbarriere") freigesetzt. Gelangen diese in die systemische Blutzirkulation, können sie auch andere Organe schädigen. Dementsprechend wird der Darm im Kontext der Sepsis nicht mehr als "stiller Teilhaber", sondern als "Motor des Multiorganversagens" betrachtet.

Die durch den Endotoxin-Stimulus im intestinalen Blut freigesetzten Entzündungsmediatoren können systemische inflammatorische Reaktionen des Körpers noch verstärken. Dabei kann es auch zu einer Erhöhung der Darmpermeabilität kommen. Dies hat zur Folge, dass vermehrt Endotoxine in den Organismus aufgenommen werden und diese wiederum die Entzündung weiter verstärken. Somit entsteht eine Perpetuierung des Vorganges im Sinne eines Circulus vitiosus.

Das aus den Zellwänden gramnegativer Darmbakterien nach Bakteriolyse durch Defensine, Bakteriophagen und Antikörper freigesetzte LPS stellt einen wichtigen Expositionsweg bei Patienten mit Operationen, Verbrennungen oder Verletzungen (Traumata) dar. Gastrointestinal freigesetztem LPS wird die Rolle eines pathogenen Stimulus bei Autoimmun-Krankheiten wie rheumatoider Arthritis und Asthma beigemessen. Bei Patienten mit Magen-Darm-Erkrankungen stellt das gastrointestinal freigesetzte LPS naturgemäß den wichtigsten Expositionsweg dar.

Wegen der hohen Systemtoxizität von Endotoxin wurden in der Vergangenheit zahlreiche Ansätze für dessen gezielte Entgiftung entwickelt.

Ein Ansatz ist hierbei die enzymatische Entgiftung von Endotoxin, unter anderem die Dephosphorylierung des Lipid A. So ist die Toxizität von Endotoxin an die Existenz zweier Phosphatgruppen pro Lipid A-Molekül geknüpft. Durch die Dephosphorylierung mindestens einer Phosphatgruppe im Lipid A-Anteil wird dessen Toxizität deutlich reduziert oder sogar gänzlich aufgehoben. Daher stellen Phosphatasen, also Enzyme, die in der Lage sind, den Lipid A-Anteil des Endotoxins zumindest teilweise zu dephosphorylieren, mögliche Kandidaten für die Behandlung Endotoxin-vermittelter Erkrankungen dar.

In diesem Zusammenhang wurde bereits die Verwendung der alkalischen Phosphatase (AP) vorgeschlagen. Für dieses Enzym wurde sowohl *in vitro* als auch *in vivo* die Dephosphorylierung von Endotoxin beschrieben und dementsprechend eine Verwendung zur Behandlung von Endotoxin-vermittelten Erkrankungen postuliert (z. B. WO 1995/005456 A1). In der WO2005/074978 A1 wird die Verwendung einer alkalischen Phosphatase zur Entgiftung von Endotoxin im Darm beschrieben.

In einem alternativen Ansatz wird die Acyloxyacylhydrolase (AOAH) zur Detoxifikation des Endotoxins eingesetzt. Diese hydrolysiert die Esterbindungen zwischen den Hydroxyfettsäuren und den sekundären Fettsäuren im Lipid A-Anteil. Dieser Ansatz beruht ursprünglich auf der Beobachtung, dass Lipid A-Analoga, denen die nicht-hydroxylierten Fettsäuren fehlen, eine geringere Toxizität aufweisen. Die Verwendung der AOAH zur Detoxifikation von Endotoxin ist in DE 37 51 588 T2 beschrieben.

Die Verwendung von Enzymen zur Detoxifikaton des Endotoxins ist jedoch auch mit Schwierigkeiten verbunden. So weisen die Enzyme eine relativ kurze Halbwertszeit auf und könnten als Komplex mit Endotoxin aus dem Darm resorbiert werden (nach EP 1 716 541 A1). Zudem besteht die Gefahr einer Interaktion mit Immunglobulinen oder mit immunkompetenten Zellen.

Der Erfindung liegt somit die Aufgabe zugrunde, ein Mittel zur Behandlung Endotoxin vermittelter Erkrankungen zur Verfügung zu stellen, das mindestens einen der im Stand der Technik vorhandenen Nachteile wenigstens reduziert.

Diese Aufgabe wird erfindungsgemäß durch ein Konjugat mit mindestens einem Polymer und mindestens einem Enzym gelöst, wobei das Enzym eine Phosphatase-Aktivität oder Acyloxyacylhydrolase-Aktivität aufweist. Damit kann es den Lipid A-Anteil des Endotoxins zumindest teilweise dephosphorylieren bzw. die nicht-hydroxylierten Fettsäuren abspalten und somit das Endotoxin entgiften. Das erfindungsgemäße Enzym-Polymer-Konjugat ist insbesondere zur Behandlung von Endotoxin-vermittelten Erkrankungen vorgesehen.

Durch die Dephosphorylierung des Lipid A-Anteils des Endotoxins entsteht insbesondere das Monophosphoryl-Lipid A (MPLA), durch die AOAH-Modifikation deacyliertes Lipid A. MPLA und deacyliertes Lipid A weisen im Gegensatz zum nativen Lipid A keine toxische Aktivität auf, werden aber wie dieses am TLR-4 gebunden. Eine Freisetzung von Entzündungsmediatoren aus Monozyten oder Makrophagen findet allerdings nicht mehr statt. Dadurch kann es eine Toleranz gegenüber einer weiteren Endotoxin-Exposition induzieren. Somit kann das erfindungsgemäße Enzym-Polymer-Konjugat nicht nur therapeutisch entgiftend wirken, sondern auch im Sinne einer Prophylaxe eingesetzt werden.

Das erfindungsgemäße Enzym-Polymer-Konjugat weist im Darm im Vergleich zu nativen Enzymen eine erhöhte Stabilität auf. Dies konnte durch Stabilitätsuntersuchungen unter Verwendung von Darmsaft nachgewiesen werden. Da dieses Milieu für die Aktivität der Enzyme besonders kritisch ist, ist die verbesserte Stabilität des erfindungsgemäßen Konjugats von besonderer praktischer Bedeutung.

Das erfindungsgemäße Enzym-Polymer-Konjugat ist, siehe oben, somit insbesondere für die orale Applikation am Patienten geeignet. Die orale Applikation erlaubt eine Endotoxin-Entgiftung im Gastrointestinaltrakt und kann damit Endotoxine schon zumindest teilweise vor ihrer Translokation in das umgebende Gewebe und Eindringen in den Blutkreislauf unschädlich machen.

In einer vorteilhaften Ausführungsform der Erfindung ist das Enzym-Polymer-Konjugat an einen Träger, insbesondere an einen festen Träger, gekoppelt.

Die erfindungsgemäße Kopplung des Enzym-Polymer-Konjugats an einen Träger dient vorteilhafterweise dazu, die Translokation des Enzym-Polymer-Konjugats durch die Darmwand ins Blut zu verhindern. Zudem kann die Kopplung des Enzym-Polymer-Konjugats an einen Träger dazu beitragen, einer Abnahme der Enzym-Aktivität, z.B. durch Proteolyse der Enzyme, vorzubeugen. Das trägergebundene Enzym-Polymer-Konjugat kann somit gegenüber dem "freien" Enzym-Polymer-Konjugat eine verlängerte Wirkdauer im Dünndarm aufweisen.

Zur Optimierung der Wirkung der Enzyme trägt außerdem bei, dass das trägergebundene Enzym-Polymer-Konjugat im Vergleich zum freien Enzym-Polymer-Konjugat langsamer durch den Verdauungstrakt transportiert wird. Diese verlangsamte Darmpassage bedeutet eine verlängerte Anwesenheit des Konjugats im Darmsaft und somit an dem relevanten Wirkort.

Zudem erlaubt die Kopplung des Enzym-Polymer-Konjugats an einen Träger eine möglichst hohe Enzym-Konzentration auf kleinem Raum, was eine optimierte Entgiftung auch lokal stark erhöhter Endotoxin-Konzentrationen ermöglicht.

### DEFINITIONEN

Ein "Enzym-Polymer-Konjugat" im Sinne der Erfindung umfaßt eine Kombination von mindestens einem Enzym und mindestens einem Polymer, wobei das Polymer mit dem Enzym verbunden ist, vorzugsweise über eine kovalente Bindung.

Ein "Konjugatpolymer" im Sinne der vorliegenden Erfindung ist ein Polymer, das mit dem Enzym zu einem Enzym-Polymer-Konjugat verbunden ist. Ein Polymer ist dabei jede Verbindung mit mindestens zwei Monomeren, die vorzugsweise strukturgleich oder strukturidentisch sind. Der Begriff Konjugatpolymer wird nachfolgend vor allem benutzt, um diese Polymere semantisch von den Polymeren des Trägers ("Trägerpolymere") abzugrenzen. "Trägerpolymere" sind Polymere, die im Träger enthalten sind oder diesen bilden. Das Konjugatpolymer hat vor allem die Funktion, das Enzym gegen einen Abbau im Darm zu schützen. Außerdem dient es zur Bindung an den Träger. In dieser Funktion dient es also als Linker.

Die "Phosphatase-Aktivität" gemäß der Erfindung ist die Spaltung eines Phosphorsäureesters durch Hydrolyse. Es wird als Synonym für "Dephosphorylierung" verwendet. Die Dephosphorylierung kann enzymatisch erfolgen, nämlich z.B. durch eine Phosphatase oder eine Dephosphorylase. Bei diesen Enzymen handelt es sich um "Proteine mit einer Phosphatase-Aktivität". Als Proteine mit einer Phosphatase-Aktivität gelten zum Beispiel die alkalische Phosphatase und die saure Phosphatase.

Als "alkalische Phosphatase" (AP) ist jedes Enzym zu verstehen, das unter die Enzymklassifikation EC 3.1.3.1 (gemäß IUBMB Enzymnomenklatur) fällt. Der Name alkalische Phosphatase hat sich durchgesetzt; es werden aber ebenso die folgenden Bezeichnungen verwendet: alkalische Phosphomonoesterase, Phosphomonoesterase, alkalische Phosphohydrolase oder Orthophosphor-Monoester Phosphohydrolase. Der systematische Name ist Phosphatmonoester Phosphohydrolase (alkalisches Optimum).

Für die Zwecke der Erfindung wird - sofern nichts anderes ausdrücklich angemerkt - nicht zwischen den beim Menschen oder Tieren existierenden verschiedenen AP, oder daraus abgeleiteten Derivaten oder Modifikationen differenziert. So sind beim Menschen mehr als 17 verschiedene AP bekannt. Die Isoenzyme der AP sind Glykoproteine, die von mindestens vier verschiedenen Genen kodiert werden. Die Gene exprimieren intestinale, plazentare, Keimzellen- und gewebeunspezifische AP. Knochen-, Niere- und Leber-AP entstehen durch posttranslationale Veränderungen der gewebeunspezifischen Genprodukte. Das Gen, das die Gewebe-spezifische AP kodiert, bildet zwei verschiedene Arten von messenger RNA: für die Zellen wird die tetramere AP synthetisiert, die mit der Außenfläche der Zellmembran über einen Carboxy-terminalen Glycan-Phosphatidylinositol-Anker verbunden ist. Für die Zirkulation werden Anker-freie AP-Dimere gebildet. Im Plasma gesunder Menschen kommen die AP-Isoenzyme der verschiedenen Gewebe hauptsächlich in der löslichen Form vor.

Die zu Laborzwecken gebräuchlichsten alkalischen Phosphatasen sind die Bacterial Alkaline Phosphatase (BAP) aus *E. coli,* die Calf Intestine Alkaline Phosphatase (CIAP) aus Kälberdarm und die Shrimp Alkaline Phosphatase (SAP) aus der Eismeergarnele. Auch sie werden nachfolgend unter dem Begriff "Alkalische Phosphatase" zusammengefasst, solange nicht anders angegeben. Gleiches gilt für modifizierte AP, wie sie zum Beispiel in der EP 0 441 252 A1 offenbart sind.

Unter dem Begriff "saure Phosphatasen" sind Phosphatasen zu verstehen, die ihr Aktivitätsoptimum im sauren Bereich aufweisen. Beispiele für saure Phosphatasen sind die prostataspezifische saure Phosphatase (ACP3), die niedermolekulare saure Phosphatase (ACP1), die lysosomale saure Phosphatase (ACP2), die Tartratresistente saure Phosphatase (ACP5), die Lysophosphatidylsäure-saure Phosphatase (ACP6), die testikuläre saure Phosphatase (ACPT) und die lysosomale saure Phosphatase.

Als "Acyloxyacylhydrolase" (AOAH) wird ein Enzym verstanden, das die Esterbindung zwischen der Säurefunktion einer Fettsäure und der Hydroxylgruppe einer zweiten Fettsäure, insbesondere einer 3-Hydroxyfettsäure, spaltet. Die Hydroxyfettsäuren sind dabei in Ester- oder Amidbindung an einen Endotoxin-Glucosaminylrest gebunden. Die AOAH gehört zur Enzymklassifikation 3.1.1.77.

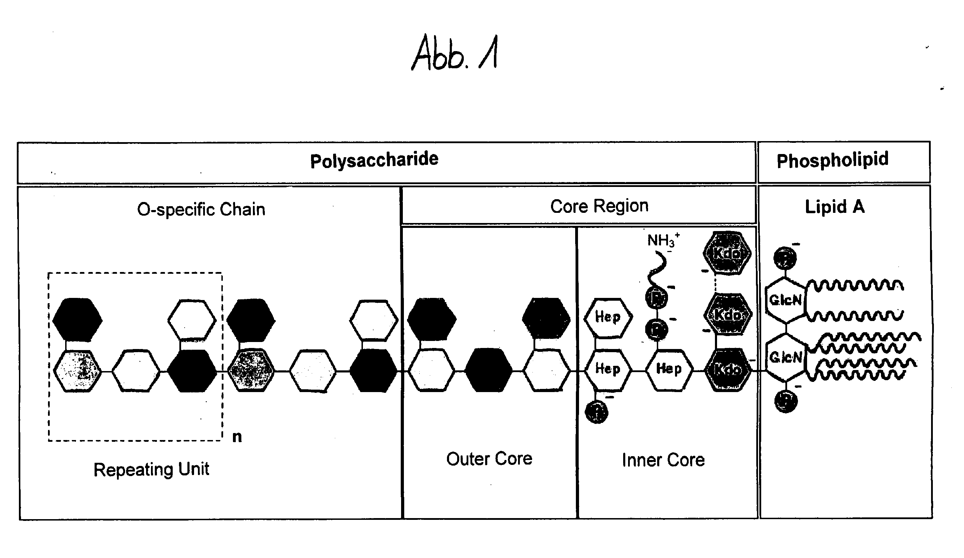

Als "Endotoxin" wird das Lipopolysaccharid (LPS) aus der Zellwand gramnegativer Bakterien verstanden, beispielsweise der folgenden Gattungen: *Neisseria, Escherichia, Salmonella, Shigella, Pseudomonas, Haemophilus* oder *Chlamydia.* Lipopolysaccharide weisen einen Polysaccharidanteil und einen Lipidanteil auf (siehe Abbildung 1). Letzterer wird als Lipid A bezeichnet. Das Lipid A ist ein Phospholipid mit einer hydrophilen Kopfgruppe. Diese ist bei Enterobakterien ein 2β-1,6-glykosidisch verknüpftes Glucosamin-Disaccharid mit mindestens zwei Phosphatgruppen an Position 1 und 4', die mit weiteren funktionellen Gruppen substituiert sein können. Weiterhin weist das Lipid A einen hydrophoben Teil auf. Dieser wird vorzugsweise von vier 3-Hydroxyfettsäuren gebildet, die primär an das Disaccharid gebunden sind, eine Kettenlänge von 10-22 C-Atomen besitzen und mit weiteren, nicht-hydroxylierten Fettsäuren (sogenannten sekundären Fettsäuren) substituiert sein können. Die inflammatorische Aktivität des Endotoxins am TLR-4 einschließlich der Wirkung auf die mit dem TLR-4 verbundenen Signalkaskade, die zur Freisetzung von Zytokinen führt, ist mit dem Lipid A assoziiert. Für die Rezeptorbindung sind die Phosphatgruppen und die sekundären Fettsäuren des Lipid A essentiell. Sofern nicht anders angegeben, wird zu Zwecken der Beschreibung der Erfindung nicht weiter zwischen Endotoxin und LPS unterschieden.

Gemäß der Erfindung ist unter dem Begriff "Entgiftung eines Endotoxins" jegliche Reduktion der biologischen Aktivität des Endotoxins, insbesondere der Wirkung auf den TLR-4 (siehe oben) zu verstehen. Die Reduktion der biologischen Aktivität erfolgt im Falle der Verwendung der Phosphatase vor allem durch die partielle oder vollständige Dephosphorylierung des Lipid A-Anteils und im Falle der Verwendung der AOAH durch die Abspaltung zumindest einer oder mehrere sekundärer Fettsäuren aus dem Lipid A-Anteil im Endotoxin.

Unter einer "Passivierung" im Sinne der Erfindung ist jegliche Behandlung der Oberfläche des Trägers zu verstehen, die die unspezifische Interaktion oder Anbindung von Darminhaltsstoffen, insbesondere von Proteinen erschwert oder unmöglich macht.

### DIE ERFINDUNG IM EINZELNEN

Das Konjugatpolymer ist ein Molekül, das mindestens zwei funktionelle Gruppen L1 und L2 aufweist, die gleich oder unterschiedlich sein können. Das Strukturelement L2 ist zur Ausbildung einer Bindung, insbesondere einer kovalenten Bindung, an das Enzym befähigt. Dabei handelt es sich bevorzugt um eine Hydroxyl- oder Carboxylgruppe, ein Succinimidylsuccinat, Succinimidylpropionat, Nitrophenylcarbonat, Tresylat, Epoxid, Aldehyd, Isocyanat oder ein Maleinimid.

Das Strukturelement L1 ist zur Ausbildung von Wasserstoffbrücken befähigt und ermöglicht die Bindung des erfindungsgemäßen Enzym-Polymer-Konjugats an die Trägerpolymeroberfläche. Weitere Untereinheiten des Konjugatpolymers können an der Ausbildung dieser Bindung beteiligt sein. L1 ist bevorzugt ein polares Wasserstoffatom, wie es beispielsweise in OH-, SH-, NH- oder PH-Bindungen vorliegt.

Bevorzugte Konjugatpolymere sind Polyalkylenoxide, besonders bevorzugt ist das Polyethylenglykol. Weiterhin können Polymere aus der folgenden Gruppe eingesetzt werden: Polyalkylenimin, Polyalkylenamin, Polyalkylensulfid oder Polyoxazoline. Die Konjugatpolymere können verschieden oder identisch sein.

In einer besonderen Ausführungsform der Erfindung ist das Konjugatpolymer ein Poly(alkylenglykol). Dies kann ausgewählt sein aus der Gruppe bestehend aus Poly(ethylenglykol) (PEG), verzweigtem PEG, Stern-PEG, Poly(propylenglykol) (PPG), Copolymeren von PEG und PPG sowie Poly(oxyethylenoxymethylen)copolymeren.

In einer ganz besonders bevorzugten Ausführungsform der Erfindung ist das Konjugatpolymer ein lineares Poly(ethylenglykol) (PEG). PEG ist aufgrund seiner guten Verträglichkeit und fehlenden Toxizität im besonderen Maße für eine Anwendung am Menschen geeignet.

Konjugatpolymere sind in der Anmeldung WO98/46648 als Moleküle mit dem Strukturelement (B) offenbart, deren entsprechende Offenbarung vollumfänglich in die vorliegende Anmeldung mit aufgenommen wird.

In einer weiteren Ausführungsform der Erfindung weist das Konjugatpolymer ein Molekulargewicht von 2 kDa bis 20 kDa, vorzugsweise von 2 bis 8 kDa, und insbesondere bevorzugt von 5 kDa auf. Dies gilt insbesondere für PEG.

Es können ein oder mehrere Konjugatpolymere an das Enzym gebunden sein. Bevorzugt sind es bis zu 20 Konjugatpolymere pro Molekül. In einer Ausführungsform sind zwischen 5 und 15 Polymere pro Enzym vorhanden. So können Enzym-Polymer-Konjugate mit einem Molekulargewicht von 80 bis 125 kDa entstehen.

Das Enzym in dem erfindungsgemäßen Enzym-Polymer-Konjugat weist in einer Ausführungsform eine Phosphatase-Aktivität auf. Bevorzugt handelt es sich um eine Phosphatase, insbesondere um eine alkalische Phosphatase. In einer besonderen Ausführungsform wird eine alkalische Säuger-Phosphatase, vorzugsweise eine humane alkalische Phosphatase eingesetzt. Diese ist vorzugsweise ausgewählt aus der Gruppe bestehend aus Dünndarm-AP, Plazenta-AP, Keimzell-AP und Gewebe-unspezifische AP, Knochen-AP, Leber-AP, Nieren-AP, Leukozyten-AP und Gallen-AP. In einer bevorzugten Ausführungsform handelt es sich bei dem Enzym um die humane plazentare alkalische Phosphatase (hpAP).

Die hpAP weist bevorzugt die folgende Aminosäuresequenz auf (SEQ ID No 1):

10         20         30         40         50         60
MLGPCMLLLL LLLGLRLQLS LGIIPVEEEN PDFWNREAAE ALGAAKKLQP AQTAAKNLII

70         80         90        100        110        120
FLGDGMGVST VTAARILKGQ KKDKLGPEIP LAMDRFPYVA LSKTYNVDKH VPDSGATATA

130        140        150        160        170        180
YLCGVKGNFQ TIGLSAAARF NQCNTTRGNE VISVMNRAKK AGKSVGVVTT TRVQHASPAG

190        200        210        220        230        240
TYAHTVNRNW YSDADVPASA RQEGCQDIAT QLISNMDIDV ILGGGRKYMF RMGTPDPEYP

250        260        270        280        290        300
DDYSQGGTRL DGKNLVQEWL AKRQGARYVW NRTELMQASL DPSVTHLMGL FEPGDMKYEI

310        320        330        340        350        360
HRDSTLDPSL MEMTEAALRL LSRNPRGFFL FVEGGRIDHG HHESRAYRAL TETIMFDDAI

370        380        390        400        410        420
ERAGQLTSEE DTLSLVTADH SHVFSFGGYP LRGSSIFGLA PGKARDRKAY TVLLYGNGPG

430        440        450        460        470        480
YVLKDGARPD VTESESGSPE YRQQSAVPLD EETHAGEDVA VFARGPQAHL VHGVQEQTFI

490        500        510        520        530
AHVMAFAACL EPYTACDLAP PAGTTDAAHP GRSVVPALLP LLAGTLLLLE TATAP

Das Enzym mit Phosphatase-Aktivität kann durch Aufreinigung biologischer Materialien (z.B. aus humaner Plazenta) oder durch rekombinante Expression gewonnen werden.

Für letzteres wird die Nukleinsäuresequenz- beispielsweise eine die SEQ. ID. NO. 1 kodierende DNA Sequenz - oder ein diese Sequenz enthaltender rekombinanter Vektor in eine geeignete Wirtszelle eingebracht, die Wirtszelle unter Bedingungen kultiviert, bei denen eine Expression der Nukleinsäuresequenz erfolgt und das Protein aus der Zelle oder dem Zellüberstand isoliert.

In einer weiteren Ausführungsform handelt es sich bei dem Enzym um eine Acyloxyacylhydrolase (AOAH).

Die Bindung des Konjugatpolymers an das Enzym kann nach dem Fachmann bekannten Verfahren erfolgen. Hierzu wird das Strukturelement L2 des Polymers verwendet (also beispielsweise Succinimidylsuccinat, Succinimidylpropionat, Nitrophenylcarbonat, Tresylat, Epoxide, Aldehyde, Isocyanate, Maleimide). So können z.B. die ε-Aminofunktionen von Lysinresten des Proteins im alkalischen Milieu (pH 8,5 - 9,5) mit PEG-Succinimidylpropionat derivatisiert werden (Roberts et al. Chemistry for peptide and protein PEGylation; Advanced Drug Delivery Reviews 54(2002), 459-476).

Weitere funktionelle Gruppen von Aminosäuren, wie z.B. die α-Aminofunktion des N-terminalen Proteinendes, die Hydroxyfunktion von Serin, Threonin und Tyrosin, die Carboxyfunktion des C-terminalen Proteinendes, von Asparaginsäure und Glutaminsäure, die Thiolfunktion von Cystein und nicht-natürliche Aminosäuren, die eine Seitenkette von ähnlicher Reaktivität tragen, sind für eine Derivatisierung zugänglich. Ebenfalls besteht z.B. die Möglichkeit der Konjugatpolymerbindung an zuvor durch Mutation eingeführten Cysteinresten oder an das mit einer reaktiven Gruppe modifizierte N-terminale Proteinende.

Die Bindung des Enzym-Polymer-Konjugats an den Träger erfolgt vorzugsweise mit Hilfe hydrophober (van der Waals) Wechselwirkungen oder hydrophiler Wechselwirkungen (Wasserstoffbrückenbindungen). Letzteres ist bevorzugt.

Diese Bindung kann spontan erreicht werden durch eine Inkubation des Trägers mit den Enzym-Polymer-Konjugaten. In einer besonderen Ausführungsform erfolgt diese Inkubation ohne polares organisches Lösungsmittel.

Die resultierende Bindung zwischen dem Enzym-Polymer-Konjugat und dem Träger ist ausreichend stabil. So bleibt die Bindung vorzugsweise in einem pH-Bereich von 2,0 bis 10,0 und bis zu Temperaturen von 70°C erhalten. Dies gilt auch für hohe Ionenstärken, z.B. 2N Glycin oder 2N Harnstofflösung. Dies bedeutet, dass sowohl unter *in vitro* als auch *in vivo*-Bedingungen eine Ablösung des Enzym-Konjugats von dem Träger in einem relevanten Umfang nicht erfolgt. Gleichwohl besteht die Möglichkeit, die Bindung zu lösen, so dass z.B. eine Regeneration des Trägermaterials unter Anwendung spezieller Verfahren erfolgen kann.

An den Träger sind vorzugsweise Konjugate bestehend aus Enzym und PEG gebunden. Vorzugsweise ist das Enzym eine alkalische Phosphatase oder eine Acyloxyacylhydrolase. Es ist aber auch möglich, dass Konjugate verschiedener Zusammensetzung an den Träger gebunden sind, so zum Beispiel Enzymkonjugate mit Polyalkylenoxid und Polyalkylenimin als Polymere oder PEGylierte AOAH zusammen mit PEGylierter AP. Es sind diesbezüglich beliebige Kombinationen möglich.

In einer weiterhin bevorzugten Variante sind auf dem Träger Konjugate mit einer Phosphatase und Konjugate mit AOAH gebunden. Bei der Phosphatase handelt es sich vorzugsweise um eine alkalische Phosphatase.

Der Träger des erfindungsgemäßen trägergebundenen Enzym-Polymer-Konjugats kann aus einem oder mehreren Polymeren aufgebaut sei, die mindestens das folgende Strukturelement A enthalten: 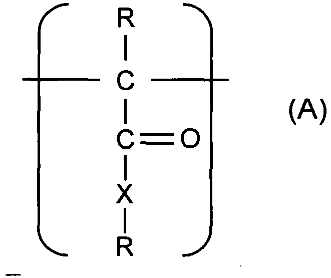 worin die Reste R gleich oder verschieden sein können und für einen beliebigen Alkyl-oder Arylrest oder ein Wasserstoffatom stehen und der fakultativ vorhandene Rest X ein O, N oder CH₂ bedeutet.

Dieses Strukturelement ist beispielsweise Teil eines Polymers der allgemeinen Formel (I): 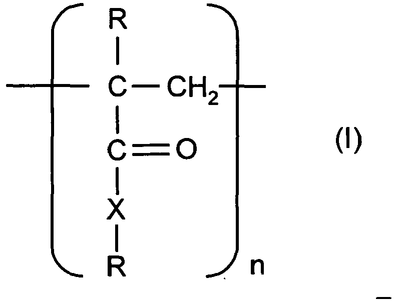 worin R einen Alkyl- oder Arylrest oder ein Wasserstoffatom bedeutet, und n den Wert 10 bis 10.000 besitzt. Der Rest X ist fakultativ und bedeutet O, N oder CH₂. Der Alkylrest kann jeder beliebige geradkettige oder verzweigtkettige, gegebenenfalls substituierte Alkylrest sein. Bevorzugt ist ein geradkettiger oder verzweigter, gegebenenfalls substituierter C₁₋₈ Alkylrest, wie beispielsweise eine Methyl-, Ethyl-, n-Propyl-, i-Propyl-, n-Butyl-, i-Butyl-, s-Butyl- oder t-Butyl-Gruppe. Beispiele für gegebenenfalls vorhandene Substituenten umfassen ein oder mehrere Halogenatom(e), z.B. Fluor-, Chlor-, Brom- oder lodatome, oder Hydroxylgruppe(n), Alkylreste wie C₁₋₆-Alkylreste oder Alkoxyreste, wie z.B. C₁₋₆-Alkoxyreste, z.B. C₁₋₃-Alkoxy, wie Methoxy- oder Ethoxygruppen, oder Thiolgruppen, wie beispielsweise C₁₋₆-Alkylthiolgruppen, z. B. C₁₋₆ -Alkylthiolgruppen, wie Methylthiol- oder Ethylthiolgruppen. Der Arylrest kann ein monocyclischer oder bicyclischer, gegebenenfalls substituierter Arylrest sein, der gegebenenfalls ein oder mehrere Heteroatome enthalten kann. Beispiele hierfür sind Phenyl-, 1- oder 2-Naphthyl-, Indenyl- oder Isoindenylreste. Gegebenenfalls können die Arylreste auch substituiert sein. Beispiele für heteroatomhaltige Arylreste sind ein C₁₋₉-Heteroarylrest, beispielsweise ein gegebenenfalls substituierter C₃₋₉-Heteroarylrest, der beispielsweise 1, 2, 3 oder mehrere Heteroatome, ausgewählt aus Sauerstoff, Schwefel oder Stickstoffatomen, enthält. Monocyclische Heteroarylreste umfassen beispielsweise Pyrolyl-, Furyl-, Thienyl-, Imidazolyl-, N-Methylimidazolyl-, N-Ethylimidazolyl-, Oxazolyl-, Disoxazolyl-, Thiazolyl-, Isothiazolyl-, Pyrazolyl-, 1,2,3-Triazolyl-, Benzofuryl-, Isobenzofuryl-, Benzothienyl-, Isobenzothienyl-, Indolyl-, Isoindolyl-, Benzimidazolyl-, Benzothiazolyl-, Chinazolinyl-, Naphthylpyridinyl-, Chinolinyl-, Isochinolinyl- und Tetrazolylgruppen.

Die Taktizität des so erhaltenen Polymeren kann beliebig sein, beispielsweise isotaktisch, heterotaktisch bzw. syndiotaktisch.

Träger mit Strukturelementen der Formel (A) sind in der Anmeldung WO1998/46648 offenbart, deren Offenbarung vollumfänglich mit in die vorliegende Anmeldung aufgenommen wird.

In einer besonderen Ausführungsform handelt es sich bei dem Trägerpolymer um ein Polyacrylat, insbesondere Polymethylmethacrylat (PMMA), ein Polyvinylester oder ein Mischpolymer oder Gemisch davon mit anderen polymerisierbaren Substanzen.

Das Trägerpolymer ist bevorzugt ein Polyalkyl(meth)acrylat (PAMA) oder ein Polyalkyl(meth)acrylat-Mischpolymer. Besonders bevorzugt ist als Polymer Polymethyl(meth)acrylat (PMMA), Polyethyl(meth)acrylat (PEMA), Polypropyl(meth)acrylat (PPMA), Polybutyl(meth)acrylat (PBMA), Polypentyl(meth)acrylat, Polyvinylacetat, Polycyclohexyl(meth)acrylat oder Polyphenyl(meth)acrylat. In einer besonders bevorzugten Ausführungsform ist das Trägerpolymer ein Mischpolymer aus PMMA und Ethylenglycoldimethacrylat (PMMA-co-EGDMA).

Erfindungsgemäß sind auch Mischungen aus beliebigen Anteilen der vorstehend genannten Polyalkyl(meth)acrylate und einem oder mehreren weiteren Polymerkomponenten, beispielsweise Polystyrol, Polyacrylnitril, Polycarbonat oder Polysulfon denkbar. Beispiele für bevorzugte Mischungen sind Polymethylmethacrylat + Polystyrol, Polymethylmethacrylat + Polyacrylnitril + Methacrylat, Polymethylmethacrylat + Polyacrylnitril + Methallylsulfonat, Polymethylmethacrylat + Polyethylenpolyvinylalkohol, Polymethylmethacrylat + Polyamid, Polymethylmethacrylat + Polysulfon.

Bevorzugt beträgt der Anteil an Polymethylmethacrylat in den Mischpolymeren mindestens 20 %, weiter bevorzugt sind 40% bzw. 60% des Trägerpolymers aus Polymethylmethacrylat.

Ebenso können Mischpolymere aus den genannten beispielhaften Polymerkomponenten hergestellt werden.

Diese Mischpolymere können nach auf dem Fachgebiet bekannten Verfahren hergestellt werden. In das Trägerpolymer können weitere Bestandteile eingelagert werden, z.B. anorganische Verbindungen wie z.B. magnetische oder paramagnetische Eisenverbindungen bzw. Eisen oder oberflächenwirksame Substanzen wie z.B. NOabspaltende Verbindungen wie z.B. Nitroprussid-Natrium. Ferner ist auch eine Dotierung mit Metallatomen wie z.B. Nickel, Zink, Gadolinium etc. möglich. Dadurch kann das System gezielt den jeweiligen therapeutischen oder prophylaktischen Erfordernissen angepasst werden. Für den therapeutischen und prophylaktischen Einsatz ist darauf zu achten, dass das Trägerpolymer physiologisch verträglich ist, d.h. mit den physiologischen Flüssigkeiten oder dem Körpergewebe keine nachteilige Wechselwirkung eingeht. Ferner kann das Interaktionssystem auch in geeignete Transportmittel z.B. Mikrosomen, Liposomen, Nanoparts etc. verpackt werden, um es leichter in den Organismus einzuschleusen.

In einer weiteren Ausführungsform handelt es sich bei dem Trägerpolymer um ein Polyacrylnitril oder ein Kopolymer oder ein Gemisch davon mit anderen polymerisierbaren Substanzen. Als weitere polymerisierbare Substanz ist hierbei ein Monomer bevorzugt, das mindestens ein von einer Carbonsäure abgeleitetes Strukturelement (A) enthält. Besonders bevorzugt ist hierbei als Monomer Methylmethacrylat oder ein Vinylester.

Der Träger kann in beliebiger Weise ausgestaltet sein, so beispielsweise als Partikel, Hohlfaser, Garn, Membran, Preßplatte, Filtermatte oder Vliesgewebe. Wenn der Träger als Partikel vorliegt, so können diese jede beliebige Form, z.B. kugelförmig, rautenförmig, hantelförmig, rhombenförmig etc. annehmen.

Es sind sphärische Partikel als Träger mit einem Durchmesser von 0,5 bis 500 µm, bevorzugt 50 bis 250 µm und weiter bevorzugt 80 bis 125 µm. Dies gilt insbesondere für PMMA als Trägerpolymer.

Bevorzugt sind die Mikropartikel porös und besitzen eine große Oberfläche. Die Poren weisen bevorzugt einen mittleren Durchmesser zwischen 130 und 330 nm, weiter bevorzugt zwischen 180 und 280 nm und besonders bevorzugt zwischen 200 und 250 nm auf.

Geeignete Mikropartikel besitzen eine spezifische Oberfläche zwischen 50 und 150 m²/g bevorzugt zwischen 75 und 125 m²/g und weiter bevorzugt zwischen 80 und 100 m²/g. Der Gesamtdurchmesser des Partikels einschließlich des Enzym-Polymer-Konjugats liegt bei unter 130 µm, vorzugsweise bei 124µm. Davon entfallen 120µm auf den Durchmesser des Partikels, während 2x20 nm auf das Enzym-Polymer-Konjugat zurückgehen.

Es kann von Vorteil sein, die Oberfläche des Trägers mit den daran gebundenen Enzym-Polymer-Konjugaten so zu behandeln, dass eine Interaktion mit Bestandteilen des Darmsafts nicht oder nur in nicht relevantem Umfang erfolgt. Anderenfalls könnten die Enzymaktivitäten beeinträchtigt werden. Diese Passivierung erfolgt vorzugsweise mit Verbindungen, die eine geringere Molekülgröße aufweisen als das Enzym-Polymer-Konjugat, so dass es möglichst zu keiner sterischen Beeinflussung der Enzymaktivität kommen kann. Ein besonders geeignetes Größenverhältnis zwischen dem Agens für die Passivierung und dem Konjugatpolymer liegt zwischen 1:5 und 1:10.

Die Passivierung erfolgt vorzugsweise durch eine Behandlung des trägergebundenen Enzym-Polymer-Konjugats mit Polyorganosiloxanen (z.B. Polydimethylsiloxan) insbesondere mit pegylierten Polyorganosiloxanen. Durch die Behandlung mit pegylierten Polysiloxanölen können die nicht von dem Enzym-Konjugatpolymer benutzten Bindungsstellen auf dem Trägerpolymer besetzt werden. Diese Bindungsstellen stehen dann für die unspezifische Interaktion mit anderen Molekülen nicht mehr zur Verfügung, d.h. alle "PEG-spezifischen" Bindungsstellen sind abgesättigt.

Die Passivierung kann durch eine Inkubation des Trägers z.B. mit Polyethylenglykol-Polypropylenglykol-Polydimethylsiloxan erfolgen. Eine Passivierung von PMMA-Partikeln mit Polysiloxanölen ist in der EP 1 282 695 B1 beschrieben. Dessen diesbezügliche Offenbarung wird für die vorliegende Erfindung in Bezug genommen. In einer besonderen Ausführungsform wird HSy115 zur Passivierung verwendet. Dabei handelt es sich um das Polyether-funktionalisierte Silikon Belsil DMC 6031 der Firma Wacker, München, BRD.

Die erfindungsgemäßen Enzym-Polymer-Konjugate (Träger gebunden oder frei) können zur Behandlung von Endotoxin-vermittelten Erkrankung eingesetzt werden. Der Begriff Behandlung umfaßt dabei jede Form der Therapie oder Prophylaxe von Endotoxin-vermittelten Erkrankungen einschließlich der Linderung entsprechender Krankheitssymptome. Dabei handelt es sich vor allem um akute oder chronische Entzündungserkrankungen, insbesondere ausgewählt aus der Gruppe bestehend aus: chronisch entzündliche Darmerkrankungen (CED), systemisches inflammatorisches Response-Syndrom (SIRS), Sepsis, septischer Schock, Multiorgandysfunktion (MODS), Autoimmunerkrankungen, insbesondere rheumatoide Arthritis, AIDS, Peritonitis, Pankreatitis, Pneumonie, Asthma, entzündliche vaskuläre Erkrankungen, Atherosklerose, Fibrose, Verbrennungen und offene Wunden.

Das trägergebundene Enzym-Polymer-Konjugat kann für die orale Anwendung beim Patienten in eine magensaftresistente Darreichungsform überführt werden. Methoden und Mittel zur Bereitstellung magensaftresistenter Verkapselungen von Wirkstoffzusammensetzungen sind dem Fachmann bekannt (z. B. Bauer, K.H., K. Lehmann, H.P. Osterwald, G. Rothgang, "Überzogene Arzneiform", Wiss. Verlagsgesellschaft mbH, Stuttgart, 1988).

### ABBILDUNGEN

- Abb. 1:: Schematischer Aufbau eines Endotoxin-Moleküls
- Abb. 2:: Grafische Darstellung der Ausbeuten bei der PEGylierung der hpAP mit Polyethylenglykolen unterschiedlicher molekularer Massen. Dargestellt ist die Gesamtausbeute (hellgrauer Balken) und der Anteil an hpAP-PEG mit höchster Aktivität.
- Abb. 3:: hpAP-PEG-Aktivitäten gegenüber den vier Substraten para-Nitrophenylphosphat (pNPP), Adenosindiphosphat (ADP), Adenosintriphosphat (ATP) und Lipopolysaccharid aus *E*. *coli* (LPS E. *coli*).
- Abb. 4:: Stabilität der PEGylierten hpAP im Darmsaft aus Jejunum, Ileum und Colon. Die Übersicht zeigt die prozentuale Aktivitätsabnahme der PEGylierten hpAP im Vergleich zum nativen Enzym. Die in der Abbildung angegebenen gerahmten Zahlen geben den prozentualen Aktivitätserhalt (= Stabilität) wieder.
- Abb. 5:: Aktivitäten der trägergebundenen und passivierten, PEGylierten hpAP gegenüber den Substraten para-Nitrophenylphosphat (pNPP) und Lipopolysaccharid aus *E*. *coli* (LPS *E*. *coli*).
- Abb. 6:: Stabilität der trägergebundenen PEGylierten hpAP im künstlichen Darmsaft nach Malik et al. Die Übersicht zeigt die prozentuale Aktivitätsabnahme für die PEG 5000 gekoppelten hpAP mit und ohne HSy115-Modifikation. Die in der Abbildung angegebenen gerahmten Zahlen geben den prozentualen Aktivitätserhalt (= Stabilität) wieder.

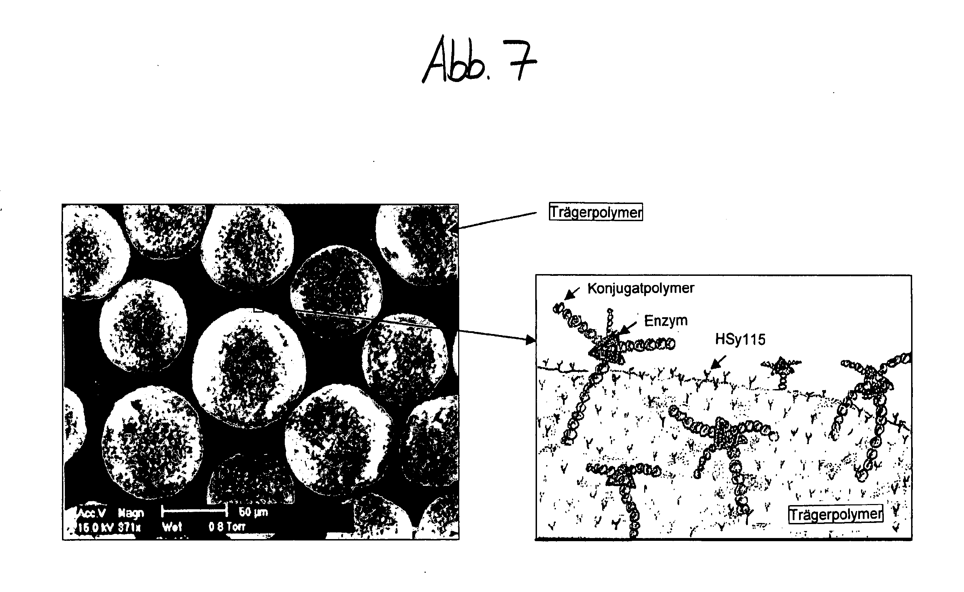

- Abb. 7:: Schematische Darstellung zum Aufbau eines trägergebundenen, passivierten Enzym-Polymer-Konjugats

### Beispiele

### I. Alkalische Phosphatase

### 1. Herstellung des alkalische Phosphatase (AP)-Polymer-Konjugats

Zu 5 mg alkalische Phosphatase werden in einem molaren Verhältnis von 1:25 11,36 mg Succiniminidyl-PEG (mPEG-SPA) 5000 (bzw. 4,54 mg mPEG-SPA 2000 bzw. 22,72 mg mPEG-SPA 10000) gegeben. Als Lösungsmittel dient ein Gemisch von 20 mM Tris-Mg/Zn-Puffer pH 8,2 und 0,5 M Borat-Puffer pH 8,5 im Verhältnis 4:1. Das Reaktionsvolumen beträgt 1 ml. Der PEGylierungsansatz wird bei 4 °C für 1 h langsam geschüttelt.

### 2. Affinitätschromatographie mit P(MMA-co-EGDMA)-Partikeln

Es werden 160 mg Polymethylmethacrylatpartikel (mittlerer Porendurchmesser: 230 nm; Partikelgröße: 80 - 125 µm) für mindestens 2 h in 1 ml 70%igen Ethanol suspendiert, danach fünfmal mit je 1 ml 20 mM Tris-Mg/Zn-/0,5 M Borat-Puffergemisch (Verhältnis 4:1) gewaschen und die PEGylierte alkalische Phosphatase aus 1. hinzu gegeben. Nach 2 h langsamen Schüttelns bei Raumtemperatur wird der Überstand abgenommen und die Partikel mit 1 ml 20 mM Tris-Mg/Zn-Puffer gewaschen. Anschließend werden die Partikel mehrmals mit je 1 ml unterschiedlich konzentrierter Methanollösung je 15 min lang geschüttelt und die Überstände vereinigt (Bsp. hpAP-PEG 5000: 2x30%igen, 6x70%igen Methanol). Es werden auf diese Weise 2 Fraktionen erhalten, wobei nur die höherprozentige Methanolfraktion (entspricht der Fraktion mit der höchsten Aktivität) für die Herstellung des AP-Polymer-Konjugates verwendet wird. Das Methanol wird bei 45 °C unter Vakuum entfernt.

### 3. Herstellung des trägergebundenen AP-Polymer-Konjugates und Passivierung der Trägeroberfläche

Es werden 95 mg Polymethylmethacrylatpartikel (mittlerer Porendurchmesser: 230 nm; Partikelgröße: 80 - 125 µm) für mindestens 2 h in 1 ml 70%igen Ethanol suspendiert, danach fünfmal mit 1 ml 20 mM Tris-Mg/Zn-Puffer gewaschen und die Fraktion der PEGylierten alkalischen Phosphatase mit der höchsten Aktivität (aus 2.) hinzugegeben. Das Konzentrationsverhältnis beträgt 6,5 µg PEGylierte alkalische Phosphatase/mg Polymethylmethacrylatpartikel. Nach 2 h langsamen Schüttelns bei Raumtemperatur wird der Überstand abgenommen, die Partikel mit 1 ml 20 mM Tris-Mg/Zn-Puffer gewaschen und 1 ml HSy115-Lösung (3,8 mg/ml) hinzugegeben. Erneut wird 2 h bei Raumtemperatur geschüttelt, der Überstand abgenommen, die Partikel mit 1 ml 20 mM Tris-Mg/Zn-Puffer gewaschen und anschließend mit 20 mM Tris-Mg/Zn-Puffer auf 1 ml aufgefüllt.

### 4. p-Nitrophenylphosphat-Assay

1 ml einer 1 mM p-Nitrophenylphosphat-Lösung in Tris-Puffer pH 9,1 wird mit 20 µl alkalische Phosphatase (1 U/ml in 20 mMTris-Mg/Zn-Puffer pH 8,2) in ein Reaktionsgefäß gegeben, gemischt und 6 min bei 37 °C inkubiert. Nach Abstoppen der Reaktion mit 0,1 ml 1 M Kaliumdihydrogenphosphat wird die Absorption der Lösung bei 405 nm vermessen.

### 5. Phosphat-Assay

nach ChandraRajan und Klein (1976) Determination of inorganic phosphorus in the presence of organic phosphorus and high concentration of proteins. Anal. Biochem. 72, 407-412.

Die Messung der AP-Aktivität gegenüber physiologischen Substraten erfolgte mit Hilfe eines Phosphatassays, bei dem nach enzymatischer Hydrolyse von Phosphatbindungen in LPS, Adenosintriphosphat (ATP) und Adenosindiphosphat (ADP) die freigesetzte Phosphatmenge quantifiziert wurde.

Substratlösungen:
1. 5 mg Lipopolysaccharid von *E.coli* O55:B5/1 ml 100 mM Tris-Puffer pH 8,2
2. 1 mg Adenosindiphosphat/1 ml 100 mM Tris-Puffer pH 8,2
3. 1 mg Adenosintriphosphat/1 ml 100 mM Tris-Puffer pH 8,2

250 µl einer Substratlösung werden mit 15 µl alkalische Phosphatase (20 U/ml in 20 mM Tris-Mg/Zn-Puffer pH 8,2) in ein Reaktionsgefäß gegeben und 1 h bei 37 °C inkubiert. 100 µl dieses Ansatzes werden anschließend mit 700 µl SDS-Acetat-Puffer (10% SDS, 0,1 M Natriumacetat) pH 4 gemischt und mit 15 µl konz. HCL versetzt. Nach abermaligem Mischen erfolgt die Zugabe von 100 µl 1%iger Ammoniummolybdat-Lösung und 100 µl 1%iger Ascorbinsäure-Lösung. Die Absorptionsmessung erfolgt bei 870 nm.

### 6. Fluorescamin-Assay

nach Böhlen et al. (1973): Fluorometric assay of proteins in the nanogram range. Arch. Biochem. Biophys. 155, 213-220

Der PEGylierungsgrad wurde durch die Reaktion des Proteins mit dem Fluoreszenzfarbstoff Fluorescamin bestimmt.

Hierbei werden 10 µl alkalische Phosphatase (entsprechen 0,68 µg Protein) mit 190 µl 0,05 M Phosphat-Puffer pH 8 versetzt. Die Reaktion wird durch Zugabe von 50 µl einer 0,03%igen Fluorescamin-Lösung in Aceton gestartet. Nach 2 min erfolgt die Fluoreszenzmessung mit der Extinktion bei 390 nm und der Emission bei 475 nm.

### 7. Darmsaftstabilität

(1) Die Messung der Stabilität erfolgte mit Schweinedarmsaft, wobei drei Darmabschnitte (Jejunum, Ileum, Colon) verwendet wurden und der Darmsaft mit Pankreatin aus Schweinepankreas angereichert wurde. Hierbei werden 300 µl des Darmsaftes aus einem Darmabschnitt mit 15 mg Pankreatin versetzt und die Reaktion durch 150 µl alkalische Phosphatase (12 U/ml in 20 mM Tris-Mg/Zn-Puffer pH 8,2) gestartet. Die Probeninkubation erfolgt im Schüttelinkubator (250 rpm) bei 37 °C über 24 h, die Probenziehung erfolgt zu den Zeitpunkten 0, 1 h, 2 h, 3 h, 22 h und 24 h. Zur Aktivitätsmessung wird der p-Nitrophenylphosphat-Assay verwendet.
(2) nach Malik et al (1976): Molecular properties of rat intestinal alkaline phosphatase. Biochim. Biophys. Acta 446, 105-114

Je 1 mg Trypsin und Chymotrypsin (aus Rinderpankreas) werden in 300 µl 0,1 M Tris-Ca-Puffer pH 8,2 gelöst und die Reaktion durch 150 µl alkalische Phosphatase (12 U/ml in 20 mM Tris-Mg/Zn-Puffer) gestartet. Die Probenlagerung erfolgt im Schüttelinkubator (250 rpm) bei 37 °C über 24 h, die Probenziehung erfolgt zu den Zeitpunkten 0, 1 h, 2 h, 3 h, 22 h und 24 h. Zur Aktivitätsmessung wird der p-Nitrophenylphosphat-Assay verwendet.

### 8. Synthese einer PEG-gekoppelten alkalischen Phosphatase

### PEGylierung:

Die PEGylierung der humanen plazentaren AP (hpAP) erfolgte mit Succinimidyl-PEG (mPEG-SPA) mit Molekulargewichten von 2000, 5000 und 10000 Da (siehe oben).

### Ergebnis:

### hpAP

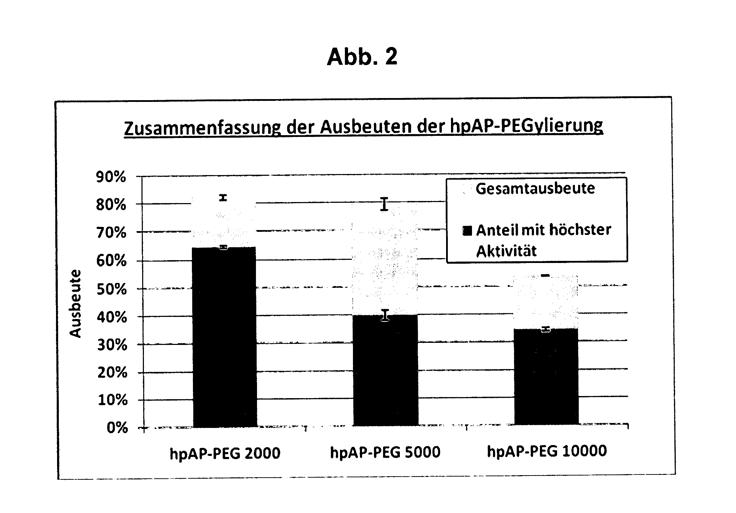

Mit der hpAP wurden die folgenden prozentualen Ausbeuten erzielt (jeweils mit Angabe des Anteils an AP mit höchster Aktivität, zur grafischen Darstellung der Ergebnisse siehe Abbildung 2):

| | Gesamtausbeute | Anteil der Fraktion mit höchster Aktivität |
|---|---|---|
| hpAP-PEG 2000 | 82,18±0,90% | 64,43±0,54% |
| hpAP-PEG 5000 | 79,38±2,03% | 39,92±1,82% |
| hpAP-PEG 10000 | 53,57±0,26% | 34,47±0,50% |

### Fazit:

Bei der hpAP nahm mit zunehmendem PEG-Molekulargewicht die Ausbeute und parallel auch der Anteil der hochaktiven AP-PEG ab. Bei einer PEGylierung mit PEG-2000 Da wurde dementsprechend die höchste Ausbeute mit 82% erzielt.

### 9. Bestimmung der Enzymaktivität der PEGylierten AP:

Die Aktivitäten der PEGylierten hpAP wurden gemäß der Methode nach ChandraRajan und Klein (siehe Beispiel 5) bzw. nach Beispiel 4 unter Verwendung von vier verschiedenen Substraten bestimmt:

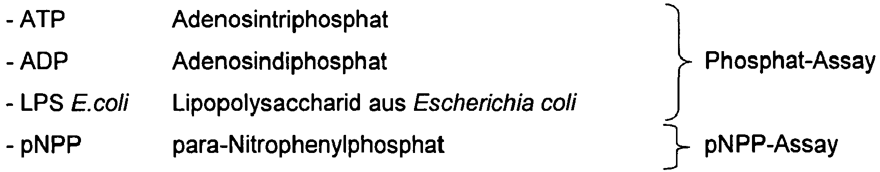

### Ergebnis:

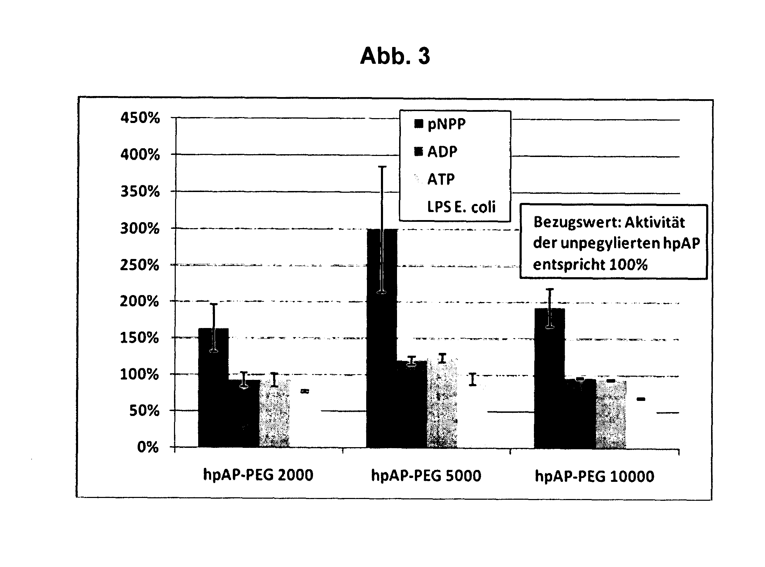

hpAP-PEG-Aktivität gegenüber den 4 unterschiedlichen Substraten (s. Abb 3):

| | pNPP | ADP | ATP | LPS E. *coli* |
|---|---|---|---|---|
| hpAP-PEG 2000 | 164,22±32,61% | 93,85±9,83% | 92,98±8,97% | 78.07±1,85% |
| hpAP-PEG 5000 | 299,44±85,15% | 120,80±5,88% | 124,06±6,12% | 95,23±8,14% |
| hpAP-PEG 10000 | 193,50±26,43% | 96,52±1,20% | 94,89±1,22% | 69,23±1,28% |

### Fazit:

Bei einem PEG-Molekulargewicht von 5000 Da war für die humane plazentare AP für alle vier Substrate die Aktivität am höchsten.

### 10. Bestimmung der Enzymaktivität der trägergebundenen PEGylierten AP:

Die Aktivitäten der trägergebundenen und passivierten PEGylierten hpAP wurden unter Verwendung der Substrate pNPP (nach Beispiel 4) und LPS aus *E.coli* gemäß der Methode nach ChandraRajan und Klein (siehe Beispiel 5) bestimmt.

### Ergebnis:

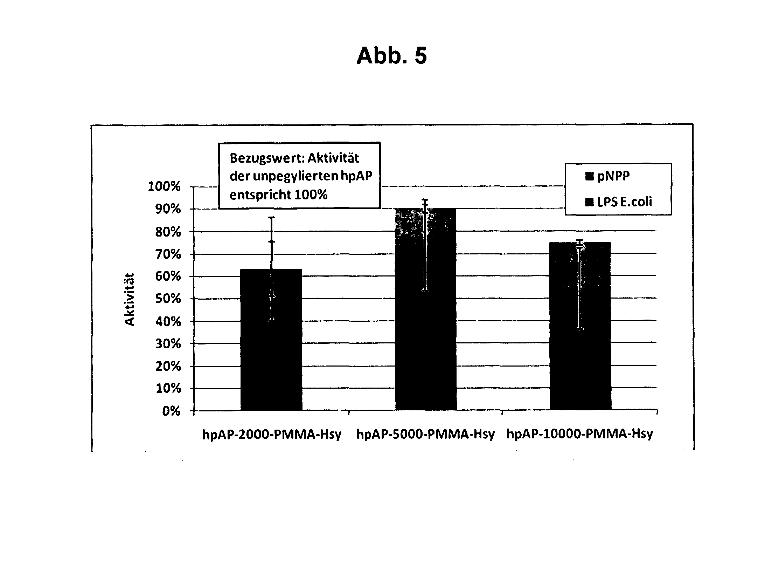

hpAP-PEG-PMMA-HSy-Aktivität gegenüber pNPP und LPS aus *E.coli* in Abhängigkeit von der PEG-Größe (s. Abb 5):

| | pNPP | LPS E. coli |
|---|---|---|
| hpAP-PEG 2000-PMMA-HSy | 62,38±12,28% | 63,19±22,92% |
| hpAP-PEG 5000-PMMA-HSy | 89,90±1,77% | 73,49±20,32% |
| hpAP-PEG 10000-PMMA-HSy | 74,98±1,12% | 54,37±18,20% |

### 11. Analyse des PEGylierungsgrades

Für die mit PEG 5000 PEGylierte AP wurde der PEGylierungsgrad mit Hilfe des Fluorescamin-Assays bestimmt (s. Beispiel 6).

### Ergebnis:

Bei der 90%-MeOH-Fraktion des hpAP-PEG 5000 (höchste Aktivität) waren im Durchschnitt 10-14 Lysine pro Protein PEGyliert (bei insgesamt 20 Lysinresten im Protein).

### 12. Stabilität der PEGylierten hpAP im Schweinedarmsaft :

Als Medium wurde ein Schweinedarmsaft aus den Darmabschnitten Jejunum, Ileum und Colon verwendet, wobei der Darmsaft mit Pankreatin aus Schweinepankreas angereichert wurde.

### Ergebnis:

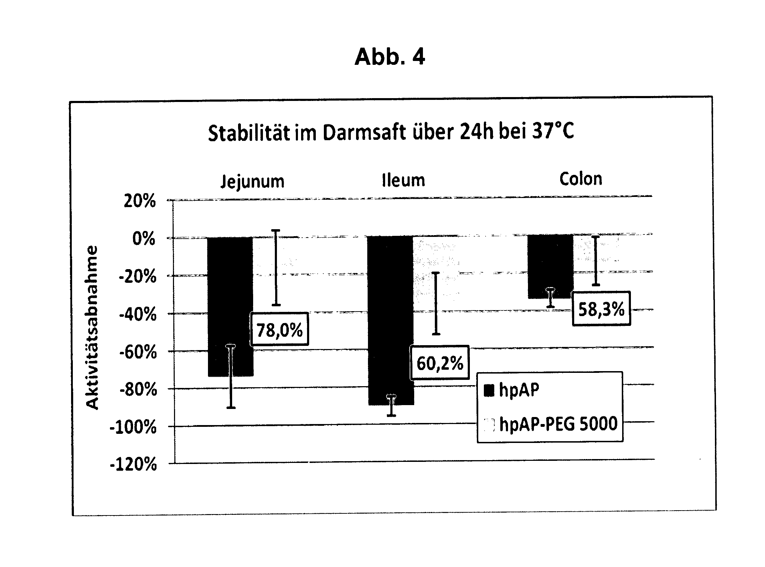

Die folgende Tabelle zeigt die prozentuale Aktivitätsabnahme der hpAP-PEG 5000 im Vergleich zur nativen hpAP (s. Abb. 4).

| | Jejunum | Ileum | Colon |
|---|---|---|---|
| hpAP | -73,91±16,53% | -90,22±5,06% | -33,52±4,45% |
| hpAP-PEG 5000 | -16,25±19,65% | -35,88±16,12% | -13,98±12,94% |

### Fazit:

Die mit PEG 5000 PEGylierte hpAP zeigt in allen drei Darmabschnitten eine deutlich höhere Stabilität als die native hpAP.

### 13. Stabilität der trägergebundenen PEGylierten hpAP (ohne und mit Passivierung) im künstlichen Darmsaft nach Malik.

### Ergebnis:

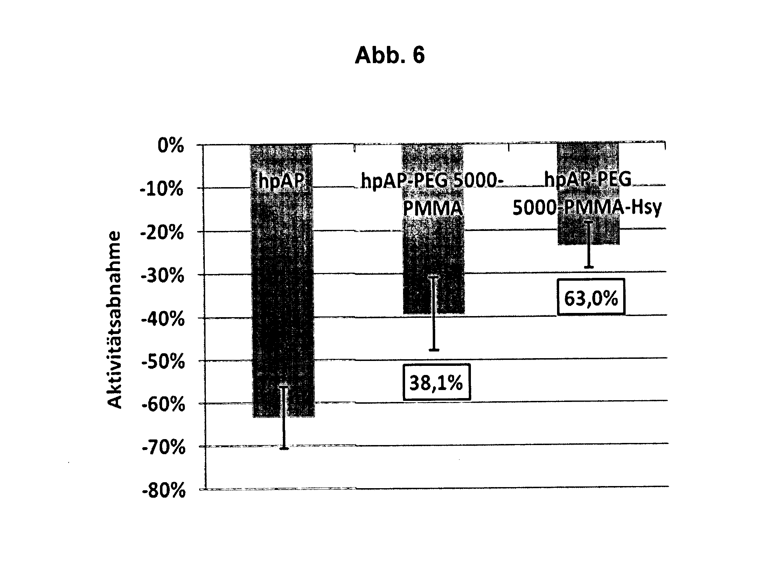

Die folgende Übersicht zeigt die prozentuale Aktivitätsabnahme der trägergebundenen/-passivierten hpAP-PEG 5000 im Vergleich zur nativen hpAP (s. Abb. 6).

| | |
|---|---|
| hpAP | -63,42 ± 7,12% |
| hpAP-PEG 5000-PMMA | -39,28 ± 8,55% |
| hpAP-PEG 5000-PMMA-HSy | -23,47 ± 5,32% |

### 14. Analyse des trägergebundenen Enzym-Polymer-Konjugate im Dottersackgefäßsystem (DSG)-Modell:

Hühnereier werden nach ~70 h Bebrütungsdauer in ex ovo-Kultur überführt. Zu diesem Zeitpunkt sind das Dottersackgefäßsystem und das Hühnerembryo in waagerechter Lage komplett ausgebreitet. Es haben sich die A. und V. vitellina mit den embryonalen Gefäßen verbunden, die kapillaren Gefäße der Dottersackmembran sind bereits deutlich ausgebildet.

Auf zwei Bereiche des Dottersackgefäßsystems werden je 4µl eines 3%igen Traganth-Gels, in dem die Proben eingebracht wurden, appliziert. Nach weiterer Bebrütung in einem Sterilinkubator bei 37°C und 85% Luftfeuchtigkeit erfolgt nach 24h die Bewertung der Vitalität der Hühnerembryonen anhand der Herzaktionen.

Die Konzentration von Endotoxin (aus *E. coli,* 055:B5) wird so gewählt, dass sie zu einer LD 80-100 führt (12,5 µg/µl). Durch Vorbehandlung dieser LPS-Menge mit dem trägergebundenen Protein-Polymer-Konjugat ist eine direkte "Entgiftung" des Endotoxins messend verfolgbar.

Im Folgenden sind die Ergebnisse für das trägergekoppelte hpAP-PEG dargestellt.

| | |
|---|---|
| PMMA-HSy (Kontrolle) | 95,5 ± 5,5% |
| hpAP-PEG 5000-PMMA-HSy | 79,1 ± 19,5% |

### II. Acyloxylacylhydrolase (AOAH)

### 1. Herstellung des AOAH-Polymer-Konjugats

Für die Herstellung der Konjugate wurde kommerziell erhältliche AOAH aus Säugerzellexpression oder selbst-exprimierte AOAH aus Insektenzellexpression verwendet.

In einem molaren Verhältnis von 1:100 wurden 50 µg AOAH und 0,15 mg Succinimidyl-PEG (mPEG-SPA) 2000 Da bzw. 0,38mg mPEG-SPA 5000 Da bzw. 0,77 mg mPEG-SPA 10000 Da in 500 µl Boratpuffer (0,1 M Borsäure; 0,1 M NaCl; pH 8,5) gelöst und für 1,5 h bei Raumtemperatur schwenkend inkubiert.

Da für die AOAH, als auch für die AP die Aktivitäten bei einem PEG-Molekulargewicht von 5000 Da am höchsten waren, wurden die folgenden Arbeiten nur mit mPEG-SPA 5000 Da durchgeführt.

Der PEG 5000-AOAH-Ansatz wurde anschließend direkt für die Bindung auf die Partikel eingesetzt.

### 2. Herstellung des trägergebundenen AOAH-Polymer-Konjugats und Passivierung der Trägeroberfäche

10 mg Polymethylmethacrylatpartikel (mittlerer Porendurchmesser 230 nm; Partikelgröße 80-125 µm) wurden zunächst in 500 µl 70%igem Methanol suspendiert und für mind. 2 h bei Raumtemperatur schwenkend inkubiert. Im Anschluss wurden die Partikel fünfmal mit Aqua bidest. gewaschen und schließlich mit 500 µl PEG 5000-AOAH-Ansatz 2 h bei Raumtemperatur geschwenkt. Nach zweimaligem Waschen der Partikel mit Aqua bidest. erfolgte die Passivierung der Partikeloberfläche durch Zugabe von 500 µl HSy115-Lösung (0,8 mg/ml). Nach erneuter Inkubation unter Schwenken für 2 h bei Raumtemperatur wurden die Partikel zweimal mit Aqua bidest. gewaschen und bei 4 °C gelagert.

### 3. Darmsaftstabilität

Die Stabilitätsuntersuchungen der AOAH erfolgten im künstlichen Darmsaft unter Verwendung von Trypsin/Chymotrypsin nach Malik et al. (1976).

Jeweils 1,67 mg Trypsin bzw. Chymotrypsin und 25 µg AOAH wurden in 500 µl 40 mM CaCl₂ gelöst und für 24 h bei 37 °C inkubiert. Die Messung der verbliebenen AOAH-Aktivität erfolgte anschließend im Glycerolassay.

### 4. Aktivitätsbestimmung der konjugierten AOAH

Die enzymatische Aktivität der AOAH wurde anhand des Glycerolassays (GA) und des *Limulus-Amöbozyten-Lysat* (LAL)-Assays nachgewiesen. In dem Glycerolassay wird das chemische Substrat 3-Palmitoyl-sn-glycerol durch die AOAH-Aktivität unter Entstehung von Glycerol gespalten, welches dann enzymatisch nachgewiesen wird.

Im LAL-Assay wird LPS durch AOAH-Aktivität deacyliert. Durch eine vergleichende Messung von "kompletten", d.h. nicht deacylierten LPS und deacylierten LPS kann dann die Aktivität der AOAH bestimmt werden.

### 4.1 Bestimmung der AOAH-Aktivität mittels Glycerolassay

Der Glycerolassay bezieht sich auf die durch AOAH-Aktivität hervorgerufene Spaltung des Substrats 3-Palmitoyl-sn-glycerol zu Glycerol (Munford & Hunter, 1992), welches daraufhin durch kommerzielle Nachweismethoden bestimmt werden kann. Für den Assay wurden 1,5 µg AOAH in 50 µl AOAH-Puffer (0,154 M NaCl; 60 mM Natriumacetat; 0,04% (v/v) Triton X-100; 1 mg/ml Rinderserumalbumin; pH 4,9) verdünnt, mit 10 µl einer 3-Palmitoyl-sn-glycerol-Lösung (50 mM in DMSO) versetzt und 4 h bei 37 °C inkubiert. Wenn eine entsprechende AOAH-Aktivität in den Proben vorhanden war, konnte das entstandene Glycerol mit Hilfe des Free Glycerol Reagent der Firma Sigma-Aldrich nachgewiesen werden. In einer Mikrotiterplatte wurden pro Well 160 µl Glycerol Reagent pipettiert und 6 µl der bereits inkubierten Probe dazugegeben. Nach einer weiteren Inkubationszeit von 5 min bei 37 °C konnten die Proben bei 540 nm vermessen werden. Zwischen Zunahme der Glycerol-Konzentration und AOAH-Aktivität konnte ein linearer Zusammenhang hergestellt werden.

### 4.2 Bestimmung der AOAH-Aktivität mittels Limulus-Amöbozyten-Lysat (LAL)-Assay

Die Messung der AOAH-Aktivität gegenüber des natürlichen Substrats Lipopolysaccharid erfolgte mittels des LAL-Assays der Firma Lonza. Um AOAH-Aktivität messen zu können, wurden zwei Ansätze mit definierter Lipopolysaccarid-Menge (750 EU) in jeweils 25 µl AOAH-Puffer (0,154 M NaCl; 60 mM Natriumacetat; 0,04% (v/v) Triton X-100; 1 mg/ml Rinderserumalbumin; pH 4,9) vorbereitet. Zu einem Ansatz wurden daraufhin weitere 25 µl AOAH-Puffer gegeben, der zweite Ansatz aber wurde mit 1,5 µg AOAH in 25 µl AOAH-Puffer aufgefüllt. Für beide Ansätze erfolgten Inkubationen von 4 h bei 37 °C und die laut Herstellerangaben durchgeführte Messung von jeweils 1 µl des Ansatzes im LAL-Assay. Das durch die AOAH detoxifizierte Lipopolysaccharid wird von den *Limulus-Gerinnungsenzymen* nicht mehr erkannt. Je größer dabei die Differenz zwischen kompletten und deacylierten Lipopolysaccharid, desto größer war auch die Aktivität der AOAH.

### 5. Aktivität der AOAH-Polymer Konjugate

Die AOAH-Polymer-Konjugate wurden wie unter 1. beschrieben unter Verwendung verschiedener Succinimidyl-PEGs (mPEG-SPA) PEGyliert. Anschließend wurde die AOAH-Aktivität mittels LAL-Assay bestimmt. Die folgende Übersicht zeigt die AOAH-Aktivität der PEGylierten Varianten anhand LAL-Assay:

| | |
|---|---|
| AOAH | 100% |
| AOAH mit mPEG-SPA 2000 Da | 45 ± 23% |
| AOAH mit mPEG-SPA 5000 Da | 50 ± 19% |
| AOAH mit mPEG-SPA 10000 Da | 0% |

### 6. Aktivität der trägergebundenen AOAH-Polymer-Konjugate

Wie unter 2. beschrieben wurde der gesamte AOAH-PEG-Ansatz zur Bindung an die PMMA-Partikel eingesetzt und anschließend die Aktivität des trägergebundenen AOAH-Polymers Partikel-PEG5000-AOAH im Vergleich zum AOAH-Polymer-Konjugats (PEG5000-AOAH) anhand LAL-Assay und GA bestimmt. Die Ergebnisse sind in der folgenden Übersicht wiedergegeben.

| | **LAL** | **GA** |
|---|---|---|
| AOAH | 100% | 100% |
| PEG 5000-AOAH | 50 ± 19% | 54 ± 15% |
| Partikel-PEG 5000-AOAH | - | 45 ± 11% |

### 7. Stabilität im künstlichen Darmsaft nach Malik et al.

Die Stabilitätsuntersuchungen der AOAH erfolgten im künstlichen Darmsaft unter Verwendung von Trypsin/Chymotrypsin nach Malik et al. (1976).

Jeweils 1,67 mg Trypsin bzw. Chymotrypsin und 25 µg AOAH wurden in 500 µl 40 mM CaCl₂ gelöst und für 24 h bei 37 °C inkubiert. Die Messung der verbliebenen AOAH-Aktivität erfolgte anschließend im GA.

Die folgende Übersicht zeigt die AOAH-Aktivitäten der AOAH, des Enzym-Polymer-Konjugats (PEG5000-AOAH) und des trägergebundenen Enzym-Polymer-Konjugats (Partikel-PEG5000-AOAH) nach Inkubation von 24 h bei 37 °C im künstl. Darmsaftmilieu anhand GA:

| | |
|---|---|
| AOAH | 80% |
| PEG 5000-AOAH | 100% |
| Partikel-PEG 5000-AOAH | 100% |

### Abkürzungen:

- AP: Alkalische Phosphatase
- AOAH: Acyloxyacylhydrolase
- hpAP: humane plazentare Alkalische Phosphatase
- LPS *E.coli*: Lipopolysaccharid aus *Escherichia coli*
- PEG: Polyethylenglykol

## Patentansprüche

1. Enzym-Polymer-Konjugat umfassend ein Enzym und mindestens ein Polymer (Konjugatpolymer), wobei das Enzym eine
a. Phosphataseaktivität oder
b. Acyloxyacylhydrolase-Aktivität zeigt
und in der Lage ist, Endotoxine zu entgiften, zur Verwendung in einem Verfahren zur Therapie oder Prophylaxe einer Endotoxin-vermittelten Erkrankung.

2. Trägergebundenes Enzym-Polymer-Konjugat umfassend mindestens i) ein Enzym, das eine Phosphataseaktivität aufweist und/oder ein Enzym, das eine Acyloxyacylhydrolase-Aktivität aufweist, und in der Lage ist, Endotoxine zu entgiften;
ii) mindestens ein Konjugatpolymer, sowie
iii) einen Träger,
wobei das Enzym über das Konjugatpolymer an dem Träger gebunden ist.

3. Enzym-Polymer-Konjugat nach Anspruch 2, **dadurch gekennzeichnet, dass** der Träger ein Trägerpolymer umfasst, das auf Basis von Monomeren aufgebaut ist, die mindestens ein von einer Carbonsäure abgeleitetes Strukturelement (A) enthalten: 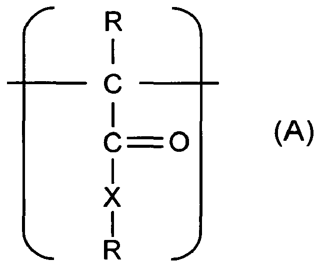 worin die Reste R gleich oder verschieden sein können und für einen beliebigen Alkyl- oder Arylrest oder ein Wasserstoffatom stehen und der fakultativ vorhandene Rest X ein O, N oder CH₂ bedeutet.

4. Enzym-Polymer-Konjugat nach Anspruch 2, **dadurch gekennzeichnet, dass** der Träger PMMA, insbesondere PMMA-co-EGDMA umfasst.

5. Enzym-Polymer-Konjugat gemäß Anspruch 2 bis 4 zur Verwendung in einem Verfahren zur Therapie oder Prophylaxe einer Endotoxin-vermittelten Erkrankung.

6. Enzym-Polymer-Konjugat nach Anspruch 1 bis 5, wobei das Konjugatpolymer ausgewählt ist aus der Gruppe (Poly)alkylenglykolen, (Poly)alkyleniminen, (Poly)alkylenaminen, (Poly)alkylensulfiden und Polyoxazolinen.

7. Enzym-Polymer-Konjugat nach einem der vorherigen Ansprüche, wobei das Konjugatpolymer ein Poly(alkylenoxid) ist, ausgewählt aus der Gruppe bestehend aus Poly(ethylenglykol) (PEG), verzweigtem PEG, Stern-PEG, Poly(propylenglykol) (PPG), Copolymeren von PEG und PPG sowie Poly(oxyethylenoxymethylen)copolymeren.

8. Enzym-Polymer-Konjugat nach einem der vorherigen Ansprüche, wobei das Konjugatpolymer ein PEG ist.

9. Enzym-Polymer-Konjugat nach einem der vorherigen Ansprüche, wobei das Konjugatpolymer ein Molekulargewicht von 2 kDa bis 20 kDa, vorzugsweise 2 bis 8 kDa, insbesondere bevorzugt 5 kDa aufweist.

10. Enzym-Polymer-Konjugat nach einem der vorherigen Ansprüche, wobei das Enzym, das Phosphataseaktivität zeigt, eine alkalische Säuger-Phosphatase, vorzugsweise eine humane alkalische Phosphatase, vorzugsweise ausgewählt aus der Gruppe bestehend aus Dünndarm-AP, Plazenta-AP, Keimzell-AP und Gewebe-unspezifische AP, Knochen-AP, Leber-AP, Nieren-AP, Leukozyten-AP und Gallen-AP.

11. Enzym-Polymer-Konjugat nach einem der vorherigen Ansprüche, wobei das Enzym, das Acyloxyacylhydrolase-Aktivität zeigt.

12. Enzym-Polymer-Konjugat nach einem der Ansprüche 2 bis 11, **dadurch gekennzeichnet, dass** die Oberfläche des Trägers passiviert ist.

13. Enzym-Polymer-Konjugat nach Anspruch 12, **dadurch gekennzeichnet, dass** die Oberfläche des Trägers mit Polyorganosiloxanen, insbesondere mit Polydimethylsiloxan, insbesondere bevorzugt mit Polyethylenglykol-Polypropylenglykol-Polydimethylsiloxan passiviert ist.

14. Enzym-Polymer-Konjugat nach einem der vorherigen Ansprüche, **dadurch gekennzeichnet, dass** die Endotoxin-vermittelte Erkrankung ausgewählt ist aus der Gruppe bestehend aus: chronisch entzündliche Darmerkrankungen (CED), Colitis ulcerosa, Morbus Crohn, systemisches inflammatorisches Response-Syndrom (SIRS), Sepsis, septischer Schock, Multiorgandysfunktion (MODS), Autoimmunerkrankungen, insbesondere rheumatoide Arthritis, AIDS, Peritonitis, Pankreatitis, Pneumonie, Asthma, entzündliche vaskuläre Erkrankungen, Atherosklerose, Fibrose und Wundheilung.

15. Enzym-Polymer-Konjugat nach Anspruch 14 zur Verwendung in einer oral applizierbaren pharmazeutischen Zusammensetzung.

16. Pharmazeutische Zusammensetzung enthaltend ein Enzym-Polymer-Konjugat gemäß einem der Ansprüche 1 bis 15.
